# EUROPEAN PATENT SPECIFICATION

(11) **EP 2 903 633 B1**
(45) Date of publication and mention of the grant of the patent: **31.01.2018**
(21) Application number: 13779637.1
(22) Date of filing: 02.10.2013
(51) Int. Cl.: A61K 38/19, A61P 35/00

(54) **CXCL3 CHEMOKINE FOR THE THERAPEUTIC TREATMENT OF MEDULLOBLASTOMA**
CXCL3 CHEMOKIN FÜR DIE THERAPEUTISCHE BEHANDLUNG VON MEDULLOBLASTOM
CHÉMOKINE CXCL3 POUR LE TRAITEMENT THÉRAPEUTIQUE DU MÉDULLOBLASTOME

(30) Priority: 02.10.2012 IT TO20120858
(43) Date of publication of application: 12.08.2015
(73) Proprietor: Consiglio Nazionale Delle Ricerche, 00185 Roma (IT)
(72) Inventor: TIRONE, Felice, 00153 Roma (IT); CAVALLARO, Sebastiano, 95030 Tremestieri Etneo (Catania) (IT); FARIOLI-VECCHIONI, Stefano, 00185 Roma (IT); MICHELI, Laura, 00137 Roma (IT); LEONARDI, Luca, 01027 Montefiascone (Viterbo) (IT); CINA', Irene, 00143 Roma (IT); CECCARELLI, Manuela, 00125 Roma (IT)
(74) Representative: Comoglio, Elena
(86) International application number: PCT/IB2013/059057
(87) International publication number: WO 2014/053999

(56) References cited:
- US-A1- 2012 064 089
- S. FARIOLI-VECCHIOLI ET AL: "Inhibition of medulloblastoma tumorigenesis by the antiproliferative and pro-differentiative gene PC3", THE FASEB JOURNAL, vol. 21, no. 9, 1 January 2007 (2007-01-01), pages 2215-2225, XP055046567, ISSN: 0892-6638, DOI: 10.1096/fj.06-7548com cited in the application
- S. FARIOLI-VECCHIOLI ET AL: "Tis21 Knock-Out Enhances the Frequency of Medulloblastoma in Patched1 Heterozygous Mice by Inhibiting the Cxcl3-Dependent Migration of Cerebellar Neurons", JOURNAL OF NEUROSCIENCE, vol. 32, no. 44, 31 October 2012 (2012-10-31), pages 15547-15564, XP055046447, ISSN: 0270-6474, DOI: 10.1523/JNEUROSCI.0412-12.2012
- JAVIER MESTAS ET AL: "The Role of CXCR2/CXCR2 Ligand Biological Axis in Renal Cell Carcinoma", THE JOURNAL OF IMMUNOLOGY, vol. 175, no. 8, 1 January 2005 (2005-01-01), pages 5351-5357, XP055046635, ISSN: 0022-1767
- TEKAMP-OLSON P ET AL: "Cloning and characterization of cDNAs for murine macrophage inflammatory protein 2 and its human homologues", THE JOURNAL OF EXPERIMENTAL MEDICINE, ROCKEFELLER UNIVERSITY PRESS, US, vol. 172, 27 August 1990 (1990-08-27), pages 911-919, XP002093659, ISSN: 0022-1007, DOI: 10.1084/JEM.172.3.911
- KRUIZINGA ROELIENE C ET AL: "Role of Chemokines and Their Receptors in Cancer", CURRENT PHARMACEUTICAL DESIGN, vol. 15, no. 29, October 2009 (2009-10), pages 3396-3416, XP009165417, ISSN: 1381-6128
- N. IIDA ET AL: "Antitumor Effect after Radiofrequency Ablation of Murine Hepatoma Is Augmented by an Active Variant of CC Chemokine Ligand 3/Macrophage Inflammatory Protein-1", CANCER RESEARCH, vol. 70, no. 16, 15 August 2010 (2010-08-15), pages 6556-6565, XP055019917, ISSN: 0008-5472, DOI: 10.1158/0008-5472.CAN-10-0096

### SEQUENCE LISTING

<110> Consiglio Nazionale delle Ricerche
<120> Chemokine for the therapeutic treatment of medulloblastoma
<130> PC1256EC
<160> 2
<170> PatentIn version 3.3
<210> 1
   <211> 107
   <212> PRT
   <213> Homo sapiens
<400> 1
   Met Ala His Ala Thr Leu Ser Ala Ala Pro Ser Asn Pro Arg Leu Leu
   1               5                   10                  15
   Arg Val Ala Leu Leu Leu Leu Leu Leu Val Ala Ala Ser Arg Arg Ala
               20                  25                  30
   Ala Gly Ala Ser Val Val Thr Glu Leu Arg Cys Gln Cys Leu Gln Thr
           35                  40                  45
   Leu Gln Gly Ile His Leu Lys Asn Ile Gln Ser Val Asn Val Arg Ser
       50                  55                  60
   Pro Gly Pro His Cys Ala Gln Thr Glu Val Ile Ala Thr Leu Lys Asn
   65                  70                  75                  80
   Gly Lys Lys Ala Cys Leu Asn Pro Ala Ser Pro Met Val Gln Lys Ile
                   85                  90                  95
   Ile Glu Lys Ile Leu Asn Lys Gly Ser Thr Asn
               100                 105
<210> 2
   <211> 1166
   <212> DNA
   <213> Homo sapiens
<400> 2
   gctccgggaa tttccctggc ccggccgctc cgggctttcc agtctcaacc atgcataaaa     60
   agggttcgcc gatcttgggg agccacacag cccgggtcgc aggcacctcc ccgccagctc    120
   tcccgcttct cgcacagctt cccgacgcgt ctgctgagcc ccatggccca cgccacgctc    180
   tccgccgccc ccagcaatcc ccggctcctg cgggtggcgc tgctgctcct gctcctggtg    240
   gccgccagcc ggcgcgcagc aggagcgtcc gtggtcactg aactgcgctg ccagtgcttg    300
   cagacactgc agggaattca cctcaagaac atccaaagtg tgaatgtaag gtcccccgga    360
   ccccactgcg cccaaaccga agtcatagcc acactcaaga atgggaagaa agcttgtctc    420
   aaccccgcat cccccatggt tcagaaaatc atcgaaaaga tactgaacaa ggggagcacc    480
   aactgacagg agagaagtaa gaagcttatc agcgtatcat tgacacttcc tgcagggtgg    540
   tccctgccct taccagagct gaaaatgaaa aagagaacag cagctttcta gggacagctg    600
   gaaaggactt aatgtgtttg actatttctt acgagggttc tacttattta tgtatttatt    660
   tttgaaagct tgtattttaa tattttacat gctgttattt aaagatgtga gtgtgtttca    720
   tcaaacatag ctcagtcctg attatttaat tggaatatga tgggttttaa atgtgtcatt    780
   aaactaatat ttagtgggag accataatgt gtcagccacc ttgataaatg acagggtggg    840
   gaactggagg gtggggggat tgaaatgcaa gcaattagtg gatcactgtt agggtaaggg    900
   aatgtatgta cacatctatt ttttatactt tttttttaaa aaaagaatgt cagttgttat    960
   ttattcaaat tatctcacat tatgtgttca acatttttat gctgaagttt cccttagaca   1020
   ttttatgtct tgcttgtagg gcataatgcc ttgtttaatg tccattctgc agcgtttctc   1080
   tttcccttgg aaaagagaat ttatcattac tgttacattt gtacaaatga catgataata   1140
   aaagttttat gaaaaaaaaa aaaaaa                                        1166

## Description

The present invention is within the field of therapeutic treatments for medulloblastoma. The invention is defined as set forth in the claims. All other embodiments are described for illustrative purposes only, and any statement that they would also be part of the invention or other, similar statements, have to be seen in the context of the application as filed and do not alter the scope of the claims. After birth, the cerebellar granule precursors (GCPs) proliferate intensively within the external granular layer (EGL), at the surface of the developing cerebellum. GCP proliferation is triggered by Sonic Hedgehog (Shh) protein, which is secreted by Purkinje neurons.

A sustained mitotic activity of GCPs increases the risk of transformation thereof. In fact, medulloblastoma, which is the most common brain tumor in children, originates from GCPs. Farioli-Vecchioli S. et al. FASEB J. 21: 2215-2225, 2007 discloses that the antiproliferative gene PC3 (Tis21/BTG2) promotes cerebellar neurogenesis by inducing GCPs to shift from proliferation to differentiation. To assess whether PC3 can prevent neoplastic transformation of GCPs and medulloblastoma development, the authors crossed transgenic mice conditionally expressing PC3 (TgPC3) in GCPs with Patched1 heterozygous mice (Ptc+/-), a model of medulloblastoma pathogenesis characterized by hyperactivation of the Sonic Hedgehog pathway. Perinatal up-regulation of PC3 in Ptc+/-/TgPC3 mice resulted in a decrease of medulloblastoma incidence of a about 40% and in a marked reduction of preneoplastic abnormalities. Moreover, overexpression of cyclin D1, hyperproliferation and defective differentiation, observed in Ptc+/-GCPs, are restored to normality in Ptc+/-/TgPC3 mice. As a whole, this indicates that PC3 may exert a tumor suppressive effect, i.e. may prevent medulloblastoma development by controlling the cell cycle and promoting differentiation of GCPs.

Medulloblastoma is a tumor that is extremely difficult to treat, in that it is particularly unresponsive to surgical and pharmacological treatments available to date.

Medulloblastoma is currently treated by surgical removal of the tumor mass, which however is not curative. Adding radiotherapy to the central nervous system at high doses may increase the recovery rate, especially when followed by chemotherapy. However, in certain cases, the side effects reported by patients are so serious that the ambition to a satisfying quality of life is compromised. Particularly, radiotherapy has a highly negative effect on the cognitive ability of pediatric patients being between the ages of two and seven, as radiations may lead to partial destruction of the neuron precursor supplies in a brain that is not yet completely formed.

Therefore, there is a dramatic need of novel therapeutic treatments for medulloblastoma which do not show the abovementioned drawbacks.

Such a need has now been fulfilled by the present inventors who found that the CXCL3 ("C-X-C motif chemokine 3") chemokine is capable of counteracting the first stage of tumorigenesis in medulloblastoma.

The CXCL3 chemokine thus represents a new therapeutic agent for treating medulloblas-toma.

CXCL3 was seen in the prior art as an oncogene (KRUIZINGA ROELIENE C et al., Current Pharmaceutical Design, vol. 15, no. 22, October 2009, pages 3396-3416) which should be suppressed in cancer treatment (US 2012/064089).

As will be described in detail in the experimental section that follows, the present inventors could identify the CXCL3 chemokine as a novel therapeutic agent for medulloblastoma, thanks to the generation of a new animal model that has been created by crossbreeding Patched1 heterozygous mice (*Ptc*^{+/-}) with mice deleted of the Tis21 gene (*Tis21*^{-/-}), which allowed to obtain *Ptc*^{+/-}*Tis21*^{-/-} double knockout mice.

Patched 1 heterozygous mice (*Ptc*^{+/-}) are a known murine medulloblastoma model (Wetmore, C. et al. Cancer Res., 60: 2239-2246, 2000; Goodrich, L. V. et al. Science (Wash. DC), 277: 1109-1113, 1997), which adequately represents the human medulloblastoma, but grows tumors at a low frequency.

Inherited or sporadic mutations in the human Patched1 gene, which encodes for Shh receptor and serves as an inhibitor of the Shh pathway in the absence of the ligand, or the absence of one Patched1 allele in murine models, are known to lead to development of medulloblastomas. This indicates that an over-activation of the Shh pathway is important in the tumor etiology. The Shh-driven proliferation of GCPs in the EGL is highest one week after birth, but three weeks later the GCPs are by then differentiated and are migrated from the EGL to the inside of the cerebellum, into the molecular and granular layers (ML; IGL), which represent their final destination. In contrast, at the same age, the EGL of Patched1 heterozygous mice (*Ptc1*^{+/-}) has GCP clusters that are still highly proliferating and that, by being different from normal GCPs in the expression profile and in the loss of the wild-type Patched1 allele, may be considered as a preneoplastic intermediate between GCPs and medulloblastoma cells. Preneoplastic GCPs may still differentiate, except for a small fraction of cells that keep on dividing. In fact, within a period of 3 to 6 months, in 15-25% of Patched1 heterozygous mice, the GCPs will develop into larger lesions and then into tumors.

Tis21, also known as PC3 or BTG2 (in mouse, rat and human being, respectively), is a transcriptional cofactor that operates in the cerebellum GCPs and in the neural progenitors of different areas of the brain, such as hippocampus and the subventricular zone, by inducing them to exit the proliferative state and differentiate. Tis21 inhibits the cell cycle in neural progenitors through direct repression of cyclin D1 promoter, and activates proneural genes through direct repression of Id3 promoter, an inhibitor of the proneural bHLH genes. The expression of this gene was found to be under-regulated in human medulloblastoma.

As mentioned previously, the present inventors crossbred *Ptc1*^{+/-} mice with *Tis21*^{-/-} mice and obtained a novel murine medulloblastoma model (*Ptc*^{+/-}*Tis21*^{-/-} double knockouts), which exhibits a number of advantageous features compared to known medulloblastoma models. In fact, the *Ptc*^{+/-}*Tis21*^{-/-} double knockouts develop medulloblastomas at a much higher frequency compared to *Ptc*^{+/-} mice (about 80%) and also exhibit a high rate of hyperplastic EGL lesions, composed of preneoplastic GCPs. Deletion of Tis21 gene in the *Ptc*^{+/-}/*Fis21*^{-/-} double knockouts does not affect the proliferation of the GCPs, but inhibits their differentiation and especially their migration outside the EGL, a feature that is not present in any other animal medulloblastoma model. As explained in further detail hereinafter, such a defect in migration represents an important step in the formation of medulloblastoma, as the GCPs, by staying longer in the EGL proliferative niche, become more prone to transformation.

The present inventors carried out comparative studies on gene expression by comparing the genome-wide expression of GCPs isolated from *Ptc*^{+/-} mice with the genome-wide expression of GCPs isolated from the *Ptc*^{+/-}/*Tis21*^{-/-} double knockout mice. Thus, the inventors found that the expression of the Cxcl3 chemokine is significantly decreased in the *Ptc*^{+/-}/*Tis21*^{-/-} double knockout mice. In *ex vivo* cerebellum slices, the inventors also observed that the addition of Cxcl3 is capable of counteracting the migration defect of the GCPs and reducing the area of the lesions, promoting the migration of preneoplastic GCPs residing in the lesions. Therefore, the neoplastic transformation process of the GCPs may be counteracted by the action of Cxcl3, which promotes the migration of preneoplastic GCPs and reduces the size of the lesions, which represent the first step of tumorigenesis.

Thus, an object of the present invention is the Cxcl3 chemokine for use in the therapeutic treatment of medulloblastoma.

The human Cxcl3 chemokine is a protein of 107 amino acids (UniProt Primary accession number: P19876), the amino acid sequence of which is known per se and shown in the sequence listing as SEQ ID NO: 1 (Homo sapiens chemokine (C-X-C motif) ligand 3 (CXCL3), mRNA, NCBI Reference Sequence: NM_002090.2).

Such a chemokine is administered, for example, to the patient in the form of a recombinant protein. Recombinant forms of the Cxcl3 chemokine are described in the state of the art and are commercially available, therefore the selection and use thereof are within the skills of a person of ordinary skill in the art.

In an alternative embodiment of the present invention, the patient is administered with an expression vector comprising a nucleotide sequence encoding the Cxcl3 chemokine.

The nucleotide sequence encoding for the human Cxcl3 chemokine is known per se and shown in the sequence listing as SEQ ID NO: 2. (Homo sapiens chemokine (C-X-C motif) ligand 3 (CXCL3), mRNA, NCBI Reference Sequence: NM_002090.2 the protein start and stop codons are underlined).

Suitable expression vectors for use in gene therapy are known and described in the state of the art, therefore the selection and use thereof are within the skills of a person of ordinary skill in the art. By way of a non-limiting example, the adeno-associated virus (AAV) vector is mentioned, the transcription of which is promoted by a cytomegalovirus/chicken beta actin promoter (CBA), which was proven to efficiently transduce a number of brain areas and is suitable because of its low associated immune reaction (Klein RL, Hamby ME, Gong Y, Hirko AC, Wang S, Hughes JA, King MA, Meyer EM. 2002. Dose and promoter effects of adeno-associated viral vector for green fluorescent protein expression in the rat brain. Exp Neurol. 176:66-74).

A further object of the present invention is a pharmaceutical composition for use in the therapeutic treatment of medulloblastoma comprising the preferably recombinant Cxcl3 chemokine, or an expression vector comprising a nucleotide sequence encoding for the Cxcl3 chemokine, in combination with pharmaceutically acceptable carriers, excipients and/or diluents.

The pharmaceutical composition of the present invention may be formulated into any dosage form suitable, for example, for administration through the oral, sublingual, buccal, rectal, intranasal, inhalation, subcutaneous, intradermal, transcutaneous, intravenous, intra-arterial, intramuscular, intraperitoneal, intrathecal or intracerebroventricular route. However, the most suitable administration route is expected to be the intrathecal one assisted by a stereotactic procedure. Of course, the selection of the suitable carriers, excipients and/or diluents is carried out according to the desired administration mode and such a selection is within the skills of a person of ordinary skill in the art. The selection of the dose of the active ingredient and the dosage regimen is also within the skills of a person of ordinary skill in the art, and the selection thereof depends on several factors, such as for instance the patient's age and the progression stage of the disease.

The following experimental section is provided merely for illustration purposes and not as a limitation of the scope of the invention as defined in the appended claims.

### EXPERIMENTAL SECTION

### Materials and Methods

### Mouse lines and genotyping

Tis21 knockout mice have been generated as described previously (Park, S. et al. Mol. Cell. Biol. 24: 10256-10262, 2004) in the C57BL/6 (B6) strain by replacing exon II of the Tis21 gene with the neomycin resistance cassette. The Patched 1 heterozygous mice (*Ptc*^{+/-}) have been generated in a CD1 background through disruption of exons 6 and 7. The *Ptc*^{-/-} embryos died before E14. By cross-breeding *Patched1* heterozygous mice with *Tis21*^{-/-} mice, double Patched1/Tis21 mutant mice were obtained, the which were interbred in order to obtain the studied genotypes. The progeny was made isogenic before starting any analysis, by further interbreeding for six or more generations.

Math1-green fluorescent protein (*Math1-*GFP) mice express GFP under the control of the Math1 enhancer.

All the animal procedures were carried out following the European Ethics Committee guidelines (86/609/CEE Directive).

### Tumor and lesion quantification and histological analysis

The mice were observed daily for medulloblastoma symptoms for a 12-month period. At the appearance of medulloblastoma symptoms (formation of a lump on the head, hunched posture, circle clockwise or anticlockwise body rotation, severe weight loss, paralysis, ruffling of fur or inactivity), the mice were sacrificed and necropsied. The cerebellum tumor was either snap frozen in liquid nitrogen for mRNA studies, or fixed in 4% paraformaldehyde by overnight immersion and then cryoprotected before sectioning in 30% sucrose in PBS-DEPC. The samples were then embedded in Tissue Teck OCT (Sakura Finetek, Tor-rance, CA, United States of America), serially sectioned and stained with hematox-ylin/eosin in order to confirm by histological analysis that the necropsied brain tissue was a medulloblastoma. The lesions were identified by visualization of proliferating GFP⁺ GCPs in asymptomatic *Ptc*^{+/-}/*Tis21*^{-/-}/*Math1-*GFP⁺ mice.

### Treatment of mice with Bromodeoxyuridine (BrdU)

The GCPs entering phase S were visualized 1 hour after BrdU injection (95 mg/kg i.p.) according to existing protocols.

### Immunohistochemistry; sample preparation, BrdU labeling, antibodies and image analysis

Histology and immunostaining of the sections, stained for multiple labeling and BrdU incorporation using fluorescent methods, were performed as described previously (Farioli-Vecchioli S. et al. FASEB J. 21: 2215-2225, 2007; Canzoniere D. et al. J. Neurosci. 24: 3355-3369, 2004). The images of the immunostained sections were obtained by laser-scanning confocal microscopy using a TCS SO5 microscope (Leica Microsystem) and were analyzed with the I.A.S. software (Delta Sistemi, Rome, Italy).

### Quantification of the cell numbers in EGL and lesions

The quantification of the proliferating, differentiating, or apoptotic cells in the EGL was performed on five non-adjacent midsagittal sections at the midpoint of the fifth, seventh and ninth folia of each section, analyzing five sections per mouse and three mice for each genotype. The number of cells in the EGL was expressed as the percentage ratio of proliferating (BrdU⁺), differentiating (NeuroD1⁺ or NeuN⁺) or apoptotic (Caspase-3⁺) cells to the total number of cells (stained with Hoechst 33258; 1 mg/ml in PBS), counted over the entire length of the EGL in each photomicrographic field, from digital images.

The BrdU⁺, NeuroD1⁺, NeuN⁺ or Caspase-3⁺ cells in the lesions were counted in the entire lesion area, defined by the presence of GFP⁺ cells, on at least five non-adjacent midsagittal sections per lesion. The inventors examined all the lesions in each one of the analyzed mice. The number of analyzed mice for each genotype and the time points are reported in the Table 1 below.

**Table 1**

| Genotype | no. (2 weeks) | no. (6 weeks) | no. (12 weeks) |
|---|---|---|---|
| *Ptc1*^{+/-}/*Tis21*^{+/+}/*Math1-*GFP | 10 | 19 | 8 |
| *Ptc1*^{+/-}/*Tis21*^{-/-}/*Math1-*GFP | 13 | 17 | 10 |

### Cell migration assa, layer areas, lesion areas and volumes

In experiments of migration from the EGL, the number of BrdU-labeled cells per square millimeter in each defined layer, i.e. EGL, molecular layer (ML) or internal granular layer (IGL), was counted in five non-adjacent midsagittal sections at the midpoint of the fifth, seventh and ninth folia. Three mice were analyzed for each genotype. Similarly, in experiments of migration from lesions, the number of BrdU-labeled cells per square millimeter of each defined layer close to the lesion (i.e. ML and IGL), was counted in three non-adjacent sagittal sections per lesion.

The planimetric measurements of EGL, ML or IGL and of the lesion area were carried out over the entire length of the layer or over the entire extension of the lesion in each photomicrographic field (5 x). The area was obtained by tracing the border of the entire layer or lesion on a digital picture captured and measured by using the I.A.S. software (Delta Sistemi).

The volume of each lesion was calculated by multiplying the average lesion area by the thickness of the section and by the number of sections wherein the lesion was detected.

### Isolation of cerebellum granule progenitors (from EGL and lesions)

The GCPs were isolated from cerebella of P7 mice following a described procedure (Wechsler-Reya, R. J. et al. Neuron 22: 103-114, 1999).

### Culture of organotypic slices (BrdU labeling, retroviral infection and immunostaining)

The slices were obtained and cultured according to the methods of Stoppini L. et al J. Neu-roshee. Methods 37: 173-182, 1991 and Polleux F. et al Science 282: 1904-1906, 1998.

The slices were cultured for 120 hours either in the presence of 100 ng/ml Cxcl3 (R&D Systems) or of the carrier alone (PBS, 0.1% BSA) and the medium was replaced every 48 hours. In order to label and then track the cells migrating from the EGL, the cultures were pulse-labeled with BrdU (10 µg/ml; Sigma) added at t0 for 18 hours. In retrovirus infection experiments with Tis21 cDNA sequence, the organotypic slices were infected by directly adding onto the slice the virus stock volume (pCAG-Tomato-Tis21 retrovirus) corresponding to 1.4 x 10⁶ TU, pulsed the following day with 10 µg/ml BrdU for 6 hours and cultured for an additional 72 hours. For each animal, one half of the cerebellum slices served as a control and were infected with the empty pCAG-Tomato virus and the other half with pCAG-Tomato-Tis21. At the end of each experiment, the slices were fixed in 4% paraformaldehyde and immunostained.

### DNA constructs and retrovirus production

The retroviral vector pCAG-IRES-tdTomato, kindly provided by Dr. Mu-ming Poo, was used to express Tis21 cDNA (i.e. the murine sequence) only in dividing GCPs. The whole open reading frame of Tis21 cDNA was cloned in the XhoI-5'/SmaI-3' sites of pCAG-IRES-tdTomato, obtaining the pCAG-IRES-tdTomato-Tis21 construct. The construct was checked by DNA sequencing.

The retroviruses were grown as previously described (Farioli-Vecchioli S. et al PLoS Biol 6 (10), and 246, 2008).

### RNA extraction, real-time RT-PCR

The total RNA, extracted from GCPs and reverse transcribed, was analyzed by real-time RT-PCR amplification, using the fluorogenic 5-nuclease assay that relies on the TaqMan probe in duplicate samples and a 7900HT system (Applied Biosystems, Foster City, CA, United States of America). The relative mRNA expression values, obtained by the comparative cycle-threshold method, were normalized to the TATA- and GAPDH-binding protein endogenous controls.

### Microarray analysis

The genome-wide expression analysis of GCPs isolated from the EGL of P7 mice was performed with Whole Mouse Genome Microarrays (Agilent Technologies, United States of America).

### Statistics

Individual comparisons among groups of the lesion frequencies and the volumes of the proliferating, differentiating, and migrating cells, and the real-time PCR values were carried out by using Student's t-test. Comparisons among groups of medulloblastoma incidence and medulloblastoma ratios were performed by the Chi-Square test. In the microarray analysis, the raw data corresponding to the signal intensity values was restricted to 1, transformed into log2, normalized to the fiftieth percentile and normalized to the baseline formed by the median of all samples. Genes with a corrected p value < 0.05 (one-way ANOVA followed by Benjamini and Hochberg False Discovery Rate test and Tukey's Post Hoc test) were considered differentially expressed.

### Results

### Tis21 ablation facilitates tumorigenesis

In order to test whether the tumorigenicity of pre-neoplastic cerebellar granule precursors (pGCPs) is affected by ablation of Tis21, the inventors assessed the frequency of medulloblastomas grown within the first year of life in Patched1 heterozygous mice (*Ptc*^{+/-}), which tend to develop a medulloblastoma spontaneously, crossbred with Tis21 knockout mice. The inventors found that while the *Ptc*^{+/-}/*Tis21*^{+/+} mice developed a medulloblastoma at a rate of 25.3%, the double mutants, *Ptc*^{+/-}/*Tis21*^{+/-} or *Ptc*^{+/-}/*Tis21*^{-/-}*,* exhibited a significantly higher medulloblastoma incidence, i.e. 80% (p = 0.000) and 75.9% (p = 0.000), respectively (Table 2). Surprisingly, however, the mean tumor latency was significantly longer in double mutants than in *Ptc*^{+/-}*lTis21*^{+/+} mice (Table 2). No tumors developed in *Ptc*^{+/-}*lTis21*^{+/+} mice (Table 2). Therefore, the ablation of one or both of the Tis21 alleles appears to strongly favor tumorigenesis in *Ptc*^{+/-} pGCPs.

**Table 2: Statistical analysis of medulloblastoma incidence and latency^{a}**

| Genotype | *Ptc*^{+/+}/ *Tis21*^{+/+} | *Ptc*^{+/+}/ *Tis21*^{-/-} | *Ptc*^{+/-}/ *Tis21*^{+/+} | *Ptc*^{+/-}/ *Tis21*^{+/-} | *Ptc*^{+/-}/ *Tis21*^{-/-} |
|---|---|---|---|---|---|
| Medulloblastoma (%) | 0 (0.0) | 0 (0.0) | 18 (25.35) | 41*(75.93) | 60*(80.00) |
| Mean latency (weeks) | -- | -- | 20.09±1.7 | 25.46±1.5^{†} | 25.57±1.3^{†} |
| No. tested mice | 40 | 42 | 71 | 54 | 75 |

| | | | | | |
|---|---|---|---|---|---|
| ^{a}The 5 genotypes relate to the progeny from crosses between Patched1 heterozygous mice and Tis21 knockout mice; all the murine genotypes are within the same genetic background, having being interbred for at least 6 generations *p < 0.0001 vs *Ptc*^{+/-}*lTis21*^{+/+} group (Chi-square test). ^{†}p < 0.05 vs *Ptc*^{+/-}*lTis21*^{+/+} group (Student's t-test). | | | | | |

To investigate such a Tis21-dependent effect, the present inventors analyzed key cell parameters of pGCPs residing within lesions, i.e. proliferation, differentiation, viability, and migration.

Patched1 heterozygous mice at P14 exhibit lesions characterized by thicker EGL regions expanded as nodular formations within cerebellar lobules and containing pGCPs with highly proliferating GCPs. These lesions are described as hyperplastic EGL remnants or focal hyperplasias if comprising more than 30 pGCPs, or diffuse hyperplasias when including more than 5000 cells. Focal hyperplasias are frequent at P14, but starting from P21 they begin to disappear, being substituted by larger diffuse hyperplasias, which indicates the occurrence of a selection of pGCPs in tumor cells.

Thus, the inventors analyzed the early hyperplasia lesions at P14, when normal GCPs in the EGL are two to three layers thick, having almost completed their migration within the IGL, whereas the remaining GCP clusters can be considered preneoplastic. The inventors also analyzed lesions at 6 and 12 weeks, when the EGL has disappeared and the external cerebellum contains foci of ectopic pGCPs.

To identify preneoplastic pGCPs in diffused and focal hyperplastic lesions, double-mutant *Ptc*^{+/-}/*Tis21*^{-/-} mice were crossed with Math1-GFP transgenic mice (Lumpkin E.A. Gene expr. patterns 3: 389-395, 2003), which carry a GFP transgene under the control of Math1, a transcription factor expressed in proliferating, normal, preneoplastic, and tumoral GCPs. The inventors verified that the progeny of *Ptc*^{+/-}*lTis21*^{-/-} double mutants crossed with Math1-GFP mice showed the same frequency and volumes of lesions and proliferative parameters of preneoplastic pGCPs as those of double mutants not crossed with Math1-GFP, as determined by BrdU labeling.

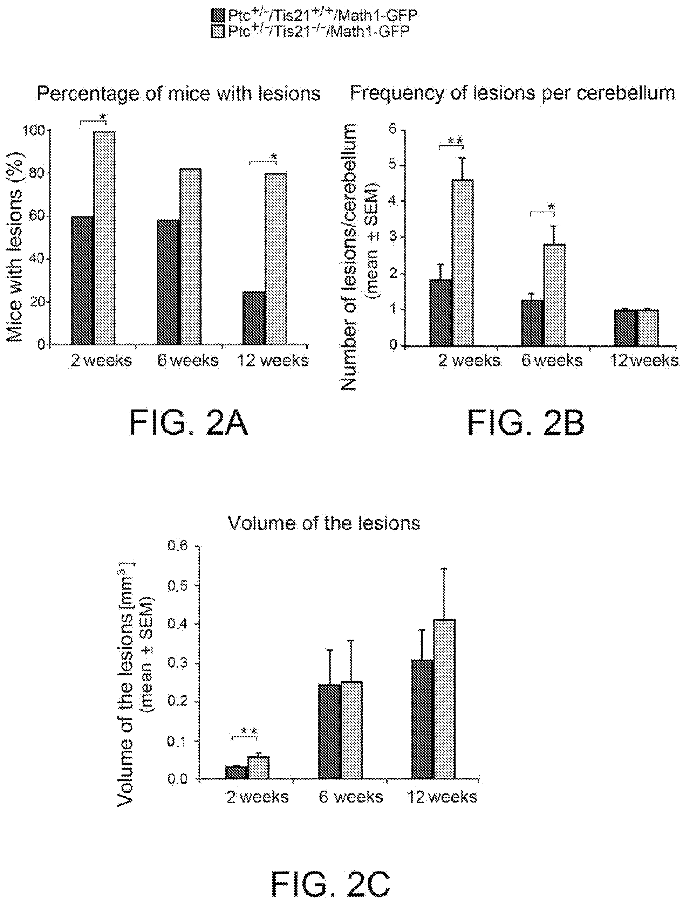

The inventors observed that the percentage of double mutant mice (*PTc*^{+/-}/*Tis21*^{-/-}/*Math1-*GFP) with diffused or focal hyperplastic lesions was significantly higher than that of Patched1 heterozygous mice (*Ptc*^{+/-}/*Tis21*^{+/+}/*Math1-*GFP) at 2 and 12 weeks after birth. Furthermore, the percentage of double mutant mice with lesions (approximately 80%) remained stable throughout the analyzed period, whereas that of *Ptc*^{+/-}/*Tis21*^{+/+}/*Math1-*GFP mice decreased rapidly from 60% to 20% within 12 weeks of birth (Figure 2A; *Ptc*^{+/-}/*Tis21*^{-/-}/*Math1-*GFP vs *Ptc*^{+/-}/*Tis21*^{+/+}/*Math1-*GFP; p = 0.02 and p = 0.03 in 2 and 12 weeks old mice, respectively). Similarly, the number of hyperplastic lesions per mice was significantly higher in double mutants than in Patched1 heterozygous mice at 2 and 6 weeks of age, although this number progressively decreased, matching, at 12 weeks, that observed in Patched1 heterozygous mice (Figure 2B; *Ptc*^{+/-}/*Tis21*^{-/-}/*Math1-*GFP vs *Ptc*^{+/-}/*Tis21*^{+/+}/*Math1-*GFP; p = 0.006 and p = 0.02 in 2 and 6 weeks old mice, respectively).

Interestingly, the percentage of mice with lesions at 12 weeks matched exactly in all genotypes the percentage of mice developing a medulloblastoma (Figure 2A; Table 2). This indicates that lesions present at 12 postnatal weeks are irreversible. In all genotypes, the inventors observed a progressive increase in lesion volumes through successive ages, with a significant increase in double mutants compared to Patched1 heterozygous mice at 2 weeks of age (Figure 2C; *Ptc*^{+/-}/*Tis21*^{-/-}/*Math1-*GFP vs *Ptc*^{+/-}/*Tis21*^{+/+}/*Math1-*GFP; p = 0.007; the number of mice analyzed in Figure 2A-C is shown in Table 2).

Collectively, these results suggested that, in *Ptc*^{+/-}/*Tis21*^{-/-} double-mutant mice, pGCPs were more prone to cluster into lesions and expand into tumors, although with a longer latency than in Patched1 heterozygous mice.

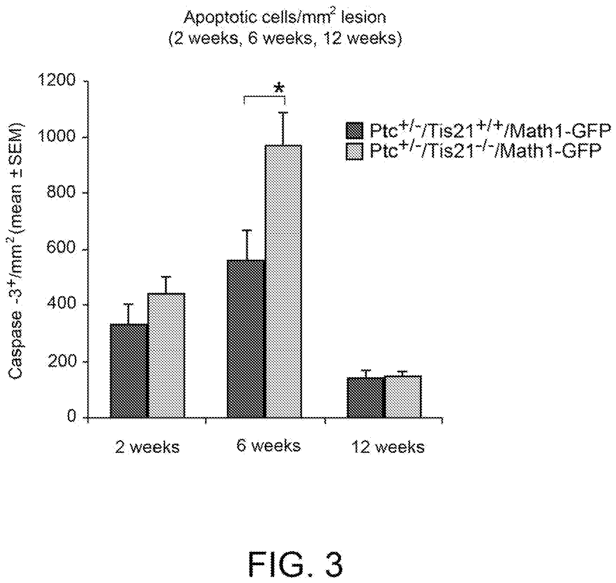

The inventors believe that the higher incidence of tumors observed in double mutants could depend on the greater frequency and persistence of lesions. To better understand this aspect, the inventors analyzed pGCPs inside the lesions, whose area was visualized by the presence of GFP⁺ cells, by measuring their proliferation rate through BrdU incorporation with a 1-hour pulse-labeling, and their differentiation, by labeling pGCPs with the neural markers NeuroD1 and NeuN. NeuroD1 labels just-differentiated postmitotic GCPs of the inner EGL and is required for their differentiation, whereas NeuN labels differentiated postmitotic granule neurons. No significant differences were detected between *Ptc*^{+/-}/*Tis21*^{-/-}/*Math1-*GFP and *Ptc*^{+/-}/*Tis21*^{+/+}/*Math1-*GFP mice in the number of either BrdU⁺, NeuroD1⁺, or NeuN⁺ cells present within the GFP⁺ lesion area, at 2, 6, or 12 weeks of age. Conversely, a significant increase of apoptosis (caspase-3⁺ cells per lesion area) was observed in *Ptc*^{+/-}/*Tis21*^{-/-}/*Math1-*GFP vs *Ptc*^{+/-}/*Tis21*^{+/+}/*Math1-*GFP mice at 6 weeks of age (Figure 3; p = 0.01). Then, the inventors measured the number and area of pGCPs migrated outside the lesions by labeling proliferating pGCPs in mice of 2 and 6 weeks of age (P14 and P42) by BrdU pulse-labeling, and following their migration to the molecular layer (ML) and the internal granule layer (IGL) over the next 42 hours or 5 days. Fewer pGCPs migrating from the lesions to the molecular layer were detected in *Ptc*^{+/-}/*Tis21*^{-/-}/*Math1-*GFP mice of 2 and 6 weeks of age already 42 hours after BrdU pulse-labeling. Such a decrease becomes significant 5 days after BrdU pulse-labeling, with a clear reduction (more than 40%) in the pGCPs migrated to both the molecular layer and the internal granule layer at 2 or 6 weeks of age (Figure 3C-E; *Ptc*^{+/-}/*Tis21*^{-/-}/*Math1-*GFP vs *Ptc*^{+/-}/*Tis21*^{+/+}/*Math1-*GFP mice, ML: p = 0.002 at 2 weeks of age, p = 0.044 at 6 weeks of age; IGL: p = 0.002 at 2 weeks of age, p = 0.02 at 6 weeks of age). Collectively, these data indicate that ablation of Tis21 impairs the migration of pGCPs outside the lesions and suggest that this defect, by delaying the disappearance of lesions from the proliferative area at the surface of the cerebellum, favors their conversion into a medulloblastoma.

### Tis21 ablation inhibits GCP differentiation and migration

Having analyzed the pGCPs inside the lesions, the inventors sought to define whether the genetic ablation of Tis21, alone (*Ptc*^{+/+}/*Tis21*^{-/-} mice) or combined with that of the Patched1 allele (*Ptc*^{+/-}/*Tis21*^{-/-} mice), affected the proliferation and differentiation of GCPs in lesion-free areas of the EGL at P7 and P14, i.e. respectively in stages preceding or corresponding to the initial formation of focal lesions.

At P7 - the age of highest expansion of GCPs - or at P14, the percentage of proliferating GCPs, identified by incorporation of BrdU through a 1 hour pulse-labeling, increases significantly only in Patched1 heterozygous mice, or in mice having wild-type Tis21 alleles or in Tis21-null mice (*Ptc*^{+/-}/*Tis21*^{+/+} or *Ptc*^{+/-}/*Tis21*^{-/-} vs *Ptc*^{+/+}/*Tis21*^{+/+}, p = 0.01 or p = 0.04 at P7 and p = 0.005 or p = 0.007 at P14, respectively; *Ptc*^{+/-}/*Tis21*^{-/-} vs *Ptc*^{+/-}/*Tis21*^{+/+}, p = 0.53 at P7 and p = 0.34 at P14).

This indicates that the absence of Tis21 does not affect the proliferation rate of GCPs, whereas the absence of one Patched1 allele, as expected, favors their division.

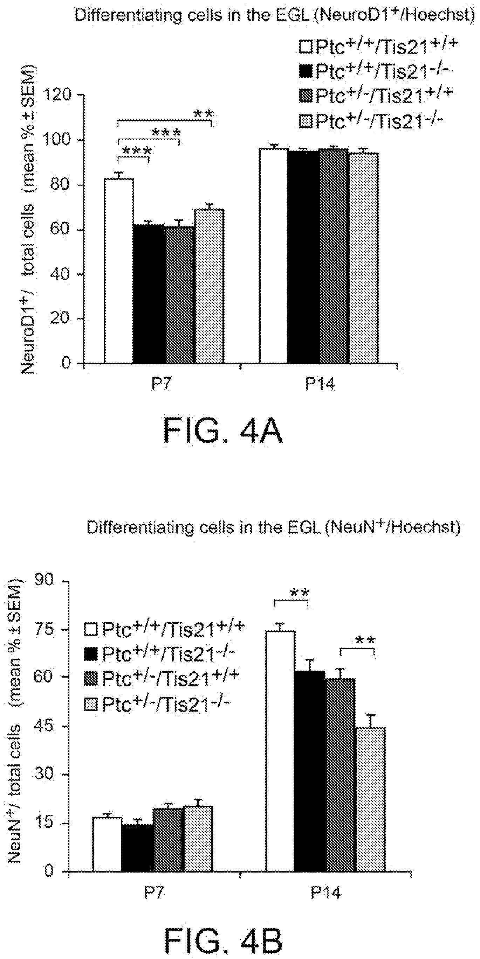

Next, the inventors analyzed the differentiation of GCPs in the EGL. At P7, ablation of Tis21, alone or in combination with one Patched1 allele, or ablation of both Tis21 and Patched1, significantly decreased to the same extent (about 25%) the percentage of NeuroD1-positive GCPs (Figure 4A; *Ptc*^{+/+}/*Tis21*^{-/-} vs *Ptc*^{+/+}/*Tis21*^{+/+}; p = 0.000; *Ptc*^{+/-}/*Tis21*^{+/+} or *Ptc*^{+/-}/*Tis21*^{-/-} vs *Ptc*^{+/+}/*Tis21*^{+/+}, p = 0.000 and p = 0.001). On the other hand, ablation of Tis21 alone, or of one Patched1 allele, induced a significant and similar decrease in GCPs positive for the late differentiation marker NeuN at P14, compared to the corresponding wild-type Tis21 controls (Figure 4B; *Ptc*^{+/+}/*Tis21*^{-/-} vs *Ptc*^{+/+}/*Tis21*^{+/+}, and *Ptc*^{+/-}/*Tis21*^{-/-} vs *Ptc*^{+/-}/*Tis21*^{+/+}, p = 0.003 and p = 0.002, respectively). Furthermore, in *Ptc*^{+/-}/*Tis21*^{-/-} double mutants, the percentage of fully differentiated GCPs (NeuN-positive at P14) was further decreased, indicating that the absence of Tis21 synergizes with Patched1 mutation in inhibiting terminal differentiation.

Together, ablation of Tis21 can impair per se both the early and late differentiation of GCPs.

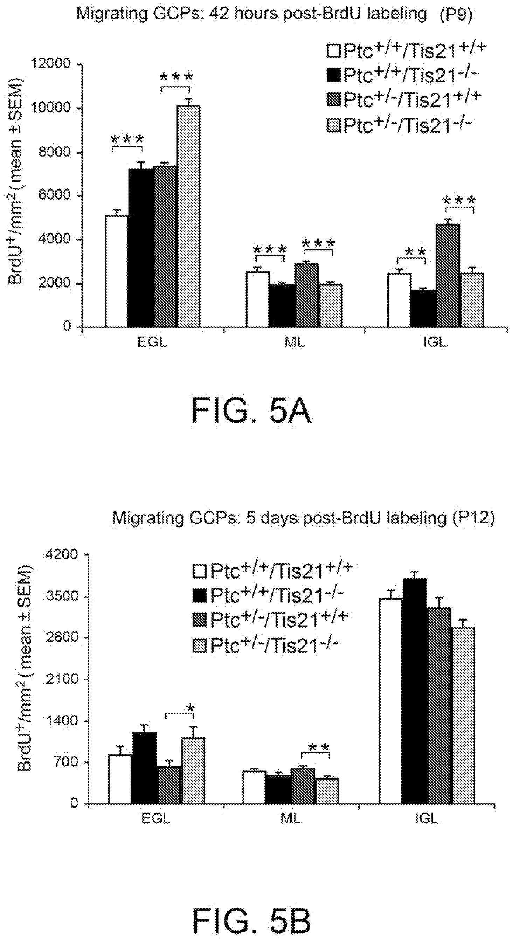

Next, the inventors measured the migration of GCPs from the EGL, by labeling them at P7 by BrdU pulse-labeling and analyzing their migration after 42 hours or after 5 days. As shown in Figure 5A, in wild-type mice up to 50% of BrdU-labeled GCPs had migrated in the ML and IGL by 42 hours after labeling. By contrast, in Tis21-null mice (*Ptc*^{+/+}/*Tis21*^{-/-}or *Ptc*^{+/-}/*Tis21*^{-/-}), a significantly lower number of BrdU-labeled GCPs had migrated in the ML and in the IGL after 42 hours (Figure 5A; *Ptc*^{+/+}/*Tis21*^{-/-} vs *Ptc*^{+/+}/*Tis21*^{+/+}, p < 0.002; or *Ptc*^{+/-}/*Tis21*^{-/-} vs *Ptc*^{+/-}/*Tis21*^{+/+}, p < 0.0001; both in ML and IGL). Consistent with the reduced migration outside the EGL, an increase of BrdU-labeled GCPs was observed in the EGL of *Tis21*^{-/-} mice, either Patched1 wild-type or heterozygous (Figure 5A; *Ptc*^{+/+}/*Tis21*^{-/-}vs *Ptc*^{+/+}/*Tis21*^{+/+}, p = 0.0000; or *Ptc*^{+/-}/*Tis21*^{-/-} vs *Ptc*^{+/-}/*Tis21*^{+/+}, p = 0.0000). The decreased migration of GCPs in the ML and IGL was specifically associated to the ablation of Tis21, as Patched1 heterozygous/Tis21 wild-type mice did not show any decrease in migration of GCPs when compared with Patched1 wild-type/Tis21 wild-type mice (*Ptc*^{+/-} /*Tis21*^{+l+} vs *Ptc*^{+/+}/*Tis21*^{+/+} in the ML, p = 0.92), or even showed an increase in migration (in the IGL). On the other hand, 5 days after labeling (at P12), the majority of BrdU-labeled GCPs had migrated outside the EGL in all groups, to reach their final destination, that is the IGL (Figure 5B). However, a significantly lower number of GCPs migrated in the ML was still detectable in double mutant Patched1 heterozygous/Tis21-null mice (Figure 5B; *Ptc*^{+/-}/*Tis21*^{-/-} vs *Ptc*^{+/-}/*Tis21*^{+/+}, p = 0.003 in the ML), suggesting that the defect in migration persisted longer if the mutation of Tis21 was associated with that of Patched1. Moreover, the inventors found no defect in Tis21-null mice, either Patched1 wild-type or heterozygous, in the organization of the Bergmann radial glia, detected by GFAP, which directs granule migration.

The inventors also assessed the survival of GCPs in the EGL at P7 and P14, and found no significant changes in the percentage of GCPs undergoing apoptosis among the four genotypes studied, as detected by positivity to Caspase-3.

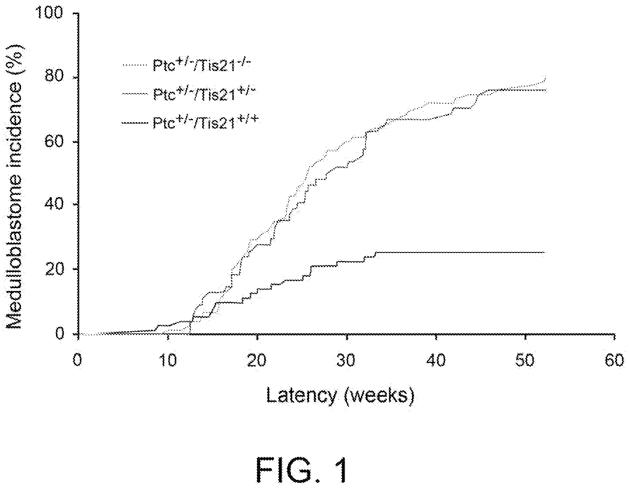

On the whole, the most evident change observed in Tis21-null, Patched1 wild-type or heterozygous mice was a migration defect of preneoplastic and normal GCPs, from lesions and EGL towards the adjacent internal layers. The migration defect of Tis21-null GCPs from the EGL was rescued late in time in Patched1 heterozygous mice, and appeared permanent in lesions, suggesting that ablation of Tis21 cooperated with Patched1 mutation in a time-dependent way. On the other hand, the differentiation defect of Tis21-null GCPs in the EGL appeared less evident compared to the migration defect, and was not detectable in lesions, suggesting that the migration defect of GCPs outside the EGL and the lesions could be of the utmost importance in the increase of tumorigenesis observed in *Ptc*^{+/-}/*Tis21*^{-/-}mice (Figure 1).

Then, in order to follow the migration process of Tis21-null GCPs in time and interfere with it, the inventors decided to analyze organotypic cerebellar slice cultures.

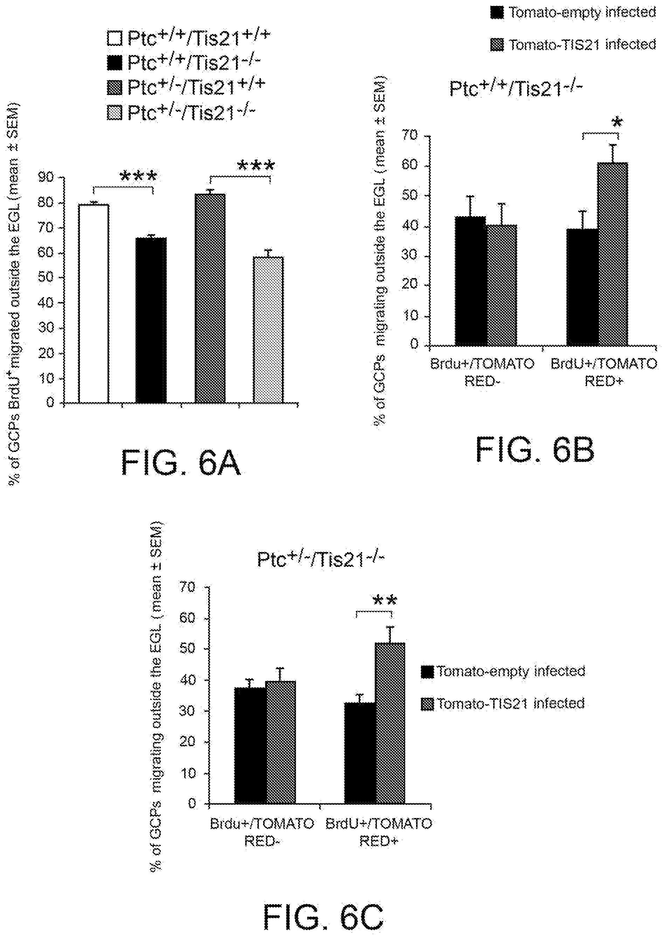

Indeed, a defective migration out of the EGL was observed in BrdU-positive GCPs of organotypic slices from Tis21-null mice, either Patched1 wild-type or heterozygous, compared with Tis21 wild-type mice (Figure 6A; *Ptc*^{+/+}/*Tis21*^{-/-} vs *Ptc*^{+/+}/*Tis21*^{+/+}, or *Ptc*^{+/-}/*Tis21*^{-/-} vs *Ptc*^{+/-}/*Tis21*^{+/+}, p = 0.0000). Moreover, when the organotypic cerebellar slices from Tis21-null mice, either Patched1 wild-type or heterozygous, were infected with a retrovirus expressing Tis21 and the Tomato red fluorescent protein, the defective migration of GCPs was significantly rescued (Figure 6B,C; Tomato-Tis21⁺ vs Tomato-empty⁺ in *Ptc*^{+/+}/*Tis21*^{-/-} mice, p = 0.02; Tomato-Tis21⁺ vs Tomato-empty⁺ in *Ptc*^{+/-}/*Tis21*^{-/-}mice, p = 0.003). This indicates that the migration defect connected to the loss of Tis21 is specific and reversible, that is to say not caused by development defects.

### Tis21 knock-out/medulloblastoma-specific gene expression

To start investigating the molecular mechanisms underlying this complex phenotype, by real-time PCR the inventors analyzed the expression levels of three main pathways associated with migration, which are known to regulate the migration of cerebellar neurons, particularly BDNF and its receptor TrkB, astrotactin1/astrotactin2, and neuregulin and its receptor Erb4.

No significant changes of these mRNAs were detected in GCPs isolated from P7 mice, either double mutant or Patched1 wild-type/Tis21-null, compared with the respective Tis21 wild-type controls. Therefore, the inventors performed a genome-wide mRNA analysis by microarray in GCPs isolated from the EGL of P7 mice. The inventors compared the gene expression between Tis21-null and Tis21 wild-type mice, either in Patched1 wild-type background (*Ptc*^{+/+}/*Tis21*^{-/-} vs *Ptc*^{+/+}/*Tis21*^{+/+}) or in Patched1 heterozygous background (*Ptc*^{+/-}/*Tis21*^{-/-} vs *Ptc*^{+/-}/*Tis21*^{+/+})*.* The inventors assumed that genes whose expression was significantly modified only in the first comparison may be related to Tis21 knock-out non-tumor-specific cerebellar phenotypes, while genes modified in the second comparison (or in both comparisons) may be specifically involved in the Tis21 knock-out-dependent enhancement of medulloblastoma formation. In this way, 344 differentially expressed genes were identified, of which 179 were Tis21 knock-out-specific and 165 medulloblastoma- specific.

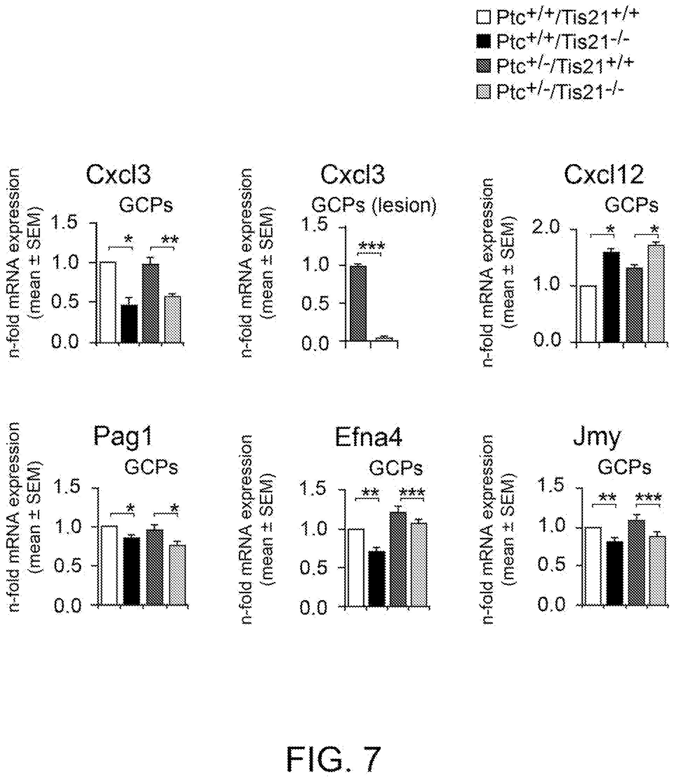

Among these, the inventors identified a few genes that showed a significantly different expression in double-mutant mice compared with Patched1 heterozygous mice, consistent with the microarray results; among these, the Cxcl3 chemokine gene was the one with the highest decrease (approximately 45%; *Ptc*^{+/-}/*Tis21*^{-/-} vs *Ptc*^{+/-}/*Tis21*^{+/+} p = 0.003). Remarkably, in pGCPs isolated from diffused lesions in 6-week-old double-mutant mice as GFP⁺ cells by FACS, Cxcl3 expression almost disappeared (95% decrease; p = 0.000; Figure 7), while the other genes did not differ.

### Cxcl3 counteracts the GCP migration defect and reduces the lesion area

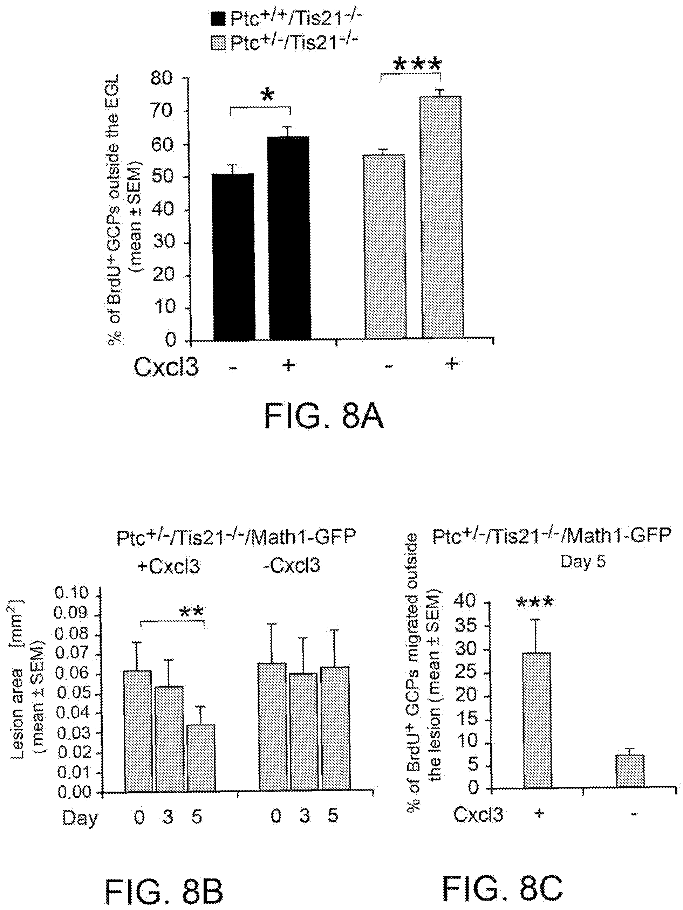

To investigate the functional implications of Cxcl3 down-regulation, the inventors tested the ability of Cxcl3 to revert the defective migration of Tis21-null GCPs. To this end, organotypic cerebellar slices from Tis21-null mice at P7, either Patched1 wild-type or heterozygous, were treated for 5 days with the Cxcl3 recombinant protein, and it was found that Cxcl3 significantly neutralized the migration defect of GCPs from the EGL, as indicated by the percentage of BrdU⁺ GCPs migrated out of the EGL (Figure 8A; with Cxcl3 vs without Cxcl3: in *Ptc*^{+/+}/*Tis21*^{-/-} mice, p = 0.001, or in *Ptc*^{+/-}/*Tis21*^{-/-} mice, p = 0.000). In particular, in double-mutant mice, Cxcl3 induced 80% of GCPs to migrate outside the EGL, attaining the basal level of migration observed in wild-type mice. Importantly, at P30, a 5-day treatment with Cxcl3 significantly reduced the lesion area in the slices (Figure 8B; Cxcl3 day 0 vs day 5, in *Ptc*^{+/-}/*Tis21*^{-/-}/*Math1-*GFP mice, p = 0.003) and, consistently, significantly counteracted the defect of migration of pGCPs (BrdU⁺-labeled) out of the lesions (Figure 8C; with Cxcl3 vs without Cxcl3, in *Ptc*^{+/-}/*Tis21*^{-/-}/*Math1-*GFP mice, p = 0.000). This indicates that the addition of exogenous Cxcl3 can rescue the Tis21-dependent defect of migration of GCPs also at a preneoplastic stage with lesions and, as a result, reduce the lesion areas.

### Discussion

The inventors have generated a new murine medulloblastoma model that enabled them to identify new mechanisms underlying the plasticity and neoplastic transformation of GCPs. In this model, the ablation of Tis21 strikingly enhances the incidence of medulloblastomas spontaneously occurring in Patched1 heterozygous mice (from 25% to 80%). Such an increase arises from the higher frequency with which Tis21-null GCPs become preneoplastic in early postnatal life and cluster in the EGL, to form focal and diffused hyperplastic lesions that will develop after 12 weeks of age into medulloblastomas. The most obvious change observed in GCPs lacking Tis21 is a remarkable delay in their migration from the EGL and the lesions to the neighboring molecular and internal granular layers. Such an effect is unique to Tis21-null mutation, given that Patched1 heterozygosity by itself does not affect GCP migration, and is associated with a delay of GCP differentiation, whereas no changes are observed in their proliferation rate either in the EGL or in the lesions. Obviously, Tis21 gene is required for migration and terminal differentiation of GCPs. GCPs were shown to proliferate when exposed to the microenvironment of the EGL proliferative niche, and exit the cell cycle when migrating away from this microenvironment. The proliferative stimulus in the EGL is Shh, a strong mitogen produced by Purkinje cells. The delay in their migration outside the EGL, detectable in Tis21-null GCPs, either Patched1 heterozygous or wild-type, causes their prolonged permanence in the EGL and thus exposure to the proliferative action of Shh. This may result in the higher frequency of lesions and higher incidence of medulloblastomas observed in the double-mutant genotype (*Ptc*^{+/-}/*Tis21*^{-/-})*.* It appears that, in *Ptc*^{+/-}/*Tis21*^{-/-} double-mutant mice, tumorigenesis follows two steps: i) at 2 weeks, when the EGL is still present, i.e. since the early postnatal stages, the percentage of double-mutant mice with lesions as well as the number of lesions per cerebellum increase significantly compared to Patched1 heterozygous/Tis21 wild-type mice (*Ptc*^{+/-}/*Tis21*^{+/+}); ii) the higher number of double mutant mice with lesions persists until 12 weeks of age, whereas at this age the percentage of Patched1 heterozygous/Tis21 wild-type mice (*Ptc*^{+/-}/*Tis21*^{+/+}) with lesions decreases; in addition, the percentage of mice with lesions at 12 weeks of age is in total agreement with that of mice developing medulloblastoma. Overall, this suggests that ablation of Tis21 in Patched1 heterozygous mice, by causing since the early postnatal period an extended exposure of dividing GCPs in the EGL area to the transforming effect exerted by Shh, may favor their preneoplastic transformation and an additional increase of tumorigenesis after 12-15 weeks of age, followed by the reaching of a plateau in the medulloblastoma incidence curve. This may also explain the longer average latency of medulloblastoma onset in double mutant mice. The inventors cannot exclude, however, that a component of such longer average latency may be a reduced diffusion of tumor GCPs, and thus a reduced tumor expansion.

The data obtained by the inventors indicated that, in Tis21-null mice, no change occurs in the expression of genes of known pathways regulating migration of cerebellar neurons, such as BDNF/TrkB, astrotactin1/astrotactin2, and neuregulin/Erb4. However, a genome-wide expression analysis of GCPs isolated from mice at P7 showed that in double-mutant mice several other genes involved in cell migration or differentiation exhibited a decrease in mRNA expression.

In particular, the inventors found that Cxcl3 showed a strong decrease of expression in GCPs, and even more strikingly in lesions of double-mutant Patched1 heterozygous/Tis21-null mice, compared to Patched1 heterozygous/Tis21 wild-type mice. As Cxcl3 reduces the lesion areas by inducing the migration of pGCPs outside the lesions, it can be speculated that this migration-promoting action of Cxcl3 may induce pGCPs to exit from the neoplastic program.

Together, these findings support the hypothesis proposed by the present inventors of a migration defect as the cause of the enhancement of medulloblastoma tumorigenesis following Tis21 ablation.

Moreover, Cxcl3 chemokine appears to be a promising new target for medulloblastoma therapy, being able to rescue this complex Tis21-dependent defective phenotype.

## Claims

1. Cxcl3 chemokine for use in the therapeutic treatment of medulloblastoma.

2. Cxcl3 chemokine for use according to claim 1, wherein Cxcl3 is a recombinant protein.

3. Cxcl3 chemokine for use according to claim 1 or 2, wherein Cxcl3 consists of amino acid sequence SEQ ID NO.1.

4. Cxcl3 chemokine for use according to any of claims 1 to 3, in a human patient.

5. An expression vector comprising a nucleotide sequence encoding for Cxcl3 chemokine, for use in the therapeutic treatment of medulloblastoma.

6. The expression vector for use according to claim 5, wherein the expression vector comprises a promoter sequence and a polyadenylation signal sequence.

7. The expression vector for use according to claim 6, wherein the encoding nucleotide sequence is SEQ ID NO.2.

8. A pharmaceutical composition for use in the therapeutic treatment of medulloblastoma, comprising a Cxcl3 chemokine according to any of claims 1 to 3 and/or an expression vector according to any of claims 5 to 7, and a pharmaceutically acceptable carrier, excipient(s) and/or diluent.

9. The pharmaceutical composition for use according to claim 8, which is in a form suitable for oral, sublingual, buccal, rectal, intranasal, inhalation, subcutaneous, intradermal, transcutaneous, intravenous, intra-arterial, intramuscular, intraperitoneal, intrathecal or intracerebroventricular administration.

10. The pharmaceutical composition for use according to claim 8 or 9, in the treatment of a human patient.

## Patentansprüche

1. Cxcl3-Chemokin zur Verwendung in der therapeutischen Behandlung eines Medulloblastoms.

2. Cxcl3-Chemokin zur Verwendung nach Anspruch 1, wobei Cxcl3 ein rekombinantes Protein ist.

3. Cxcl3-Chemokin zur Verwendung nach Anspruch 1 oder 2, wobei Cxcl3 aus der Aminosäuresequenz SEQ ID NO.1 besteht.

4. Cxcl3-Chemokin zur Verwendung nach einem der Ansprüche 1 bis 3 in einem menschlichen Patienten.

5. Expressionsvektor, der eine Nukleotidsequenz umfasst, die ein Cxcl3-Chemokin kodiert, zur Verwendung in der therapeutischen Behandlung eines Medulloblastoms.

6. Expressionsvektor zur Verwendung nach Anspruch 5, wobei der Expressionsvektor eine Promotorsequenz und eine Polyadenylierungssignalsequenz umfasst.

7. Expressionsvektor zur Verwendung nach Anspruch 6, wobei die kodierende Nukleotidsequenz SEQ ID NO.2 ist.

8. Pharmazeutische Zusammensetzung zur Verwendung in der therapeutischen Behandlung eines Medulloblastoms, umfassend ein Cxcl3-Chemokin nach einem der Ansprüche 1 bis 3 und/oder einen Expressionsvektor nach einem der Ansprüche 5 bis 7, und (ein(en)) pharmazeutisch verträgliche(n(s)) Träger, Hilfsstoff(e) und/oder Verdünnungsmittel.

9. Pharmazeutische Zusammensetzung zur Verwendung nach Anspruch 8, die in einer Form vorliegt, die für eine orale, sublinguale, bukkale, rektale, intranasale, einatmende, subkutane, intradermale, transkutane, intravenöse, intraarterielle, intramuskuläre, intraperitoneale, intrathekale oder intracerebroventrikuläre Verabreichung geeignet ist.

10. Pharmazeutische Zusammensetzung zur Verwendung nach Anspruch 8 oder 9 in der Behandlung eines menschlichen Patienten.

## Revendications

1. Chimiokine Cxcl3 à utiliser dans le traitement thérapeutique d'un médulloblastome.

2. Chimiokine Cxcl3 à utiliser selon la revendication 1, dans laquelle Cxcl3 est une protéine recombinante.

3. Chimiokine Cxcl3 à utiliser selon la revendication 1 ou 2, dans laquelle Cxcl3 consiste en la séquence d'acides aminés SEQ ID N° 1.

4. Chimiokine Cxcl3 à utiliser selon l'une quelconque des revendications 1 à 3, chez un patient humain.

5. Vecteur d'expression comprenant une séquence nucléotidique codant pour la chimiokine Cxcl3, à utiliser dans le traitement thérapeutique d'un médulloblastome.

6. Vecteur d'expression à utiliser selon la revendication 5, dans lequel le vecteur d'expression comprend une séquence promoteur et une séquence signal de polyadénylation.

7. Vecteur d'expression à utiliser selon la revendication 6, dans lequel la séquence nucléotidique codante est SEQ ID N° 2.

8. Composition pharmaceutique à utiliser dans le traitement thérapeutique d'un médulloblastome, comprenant une chimiokine Cxcl3 selon l'une quelconque des revendications 1 à 3 et/ou un vecteur d'expression selon l'une quelconque des revendications 5 à 7, et un support, excipient(s) et/ou diluant pharmaceutiquement acceptable(s).

9. Composition pharmaceutique à utiliser selon la revendication 8, qui se présente sous une forme convenant à une administration orale, sublinguale, buccale, rectale, intranasale, par inhalation, sous-cutanée, intradermique, transcutanée, intraveineuse, intra-artérielle, intramusculaire, intrapéritonéale, intrathécale ou intracérébroventriculaire.

10. Composition pharmaceutique à utiliser selon la revendication 8 ou 9, dans le traitement d'un patient humain.
